Europäisches Patentamt

European Patent Office

Office européen des brevets

(19)

(11) Numéro de publication: **0 074 984**
**B1**

(12) # FASCICULE DE BREVET EUROPEEN

(45) Date de publication du fascicule du brevet:
**07.01.88**

(51) Int. Cl.⁴: **A 61 F 13/18,** A 41 B 13/02

(21) Numéro de dépôt: **82900961.2**

(22) Date de dépôt: **19.03.82**

(86) Numéro de dépôt international:
**PCT/FR 82/00055**

(87) Numéro de publication internationale:
**WO 82/03170 (30.09.82** Gazette **82/23)**

(54) **ARTICLES D'HYGIENE POUR L'ABSORPTION DES LIQUIDES CORPORELS.**

(30) Priorité: **19.03.81 FR 8105477**

(43) Date de publication de la demande:
**30.03.83 Bulletin 83/13**

(45) Mention de la délivrance du brevet:
**07.01.88 Bulletin 88/1**

(84) Etats contractants désignés:
**AT BE CH DE GB LI LU NL SE**

(56) Documents cités:
**FR - A - 2 134 748**
**FR - A - 2 374 890**
**FR - A - 2 388 515**
**US - A - 3 814 100**

(73) Titulaire: **BEGHIN-SAY SOCIETE ANONYME,**
**F-59239 Thumeries (FR)**

(72) Inventeur: **PIGNEUL, Raymond, 2, rue des Vosges,**
**F-68320 Durrenentzen (FR)**

(74) Mandataire: **Quéré, Jean Pierre, BEGHIN-SAY Service**
**Propriété Industrielle 54, Avenue Hoche, F-75008 Paris**
**(FR)**

Il est rappelé que: Dans un délai de neuf mois à compter de la date de publication de la mention de la délivrance du brevet euro-péen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

ACTORUM AG

# Description

La présente invention se rapporte aux articles d'hygiène pour l'absorption des liquides menstruels tels que, des serviettes périodiques.

De tels articles présentent une structure stratifiée constituée d'un matelas d'absorption et de rétention des liquides (matelas de fibres cellulosiques) disposé entre une feuille perméable aux liquides en contact avec le corps humain, et une feuille extérieure imperméable aux liquides. A cette structure s'ajoutent des moyens pour assurer un bon maintien en position de l'article sur le corps.

Ainsi, dans le cas d'une serviette périodique, il est prévu, sur la face non dirigée vers le corps, une ou plusieurs lignes de colle, continues ou interrompues, pour la fixation de la serviette sur un sous-vêtement. Les lignes de colle sont disposées le plus souvent dans la partie médiane, c'est-à-dire plus près de l'axe longitudinal de la serviette que des bords de celle-ci.

Compte-tenu des différences anatomiques des utilisatrices, il n'est pas possible d'éviter les déformations importantes de la masse absorbante de la serviette. Ces déformations importantes sont préjudiciables à l'absorption des liquides menstruels, et sont la cause de fuites qui souillent la lignerie.

Les fuites sont également favorisées par le fait qu'il n'existe pas de barrage efficace dans les zones latérales de la masse absorbante.

Pour remédier à ces inconvénients, le brevet FR 2 374 890 propose une structure d'article absorbant selon laquelle la feuille extérieure imperméable aux liquides enveloppe le matelas de masse absorbante sur toute son épaisseur et est également appliquée sur les bords longitudinaux de la face du matelas dirigée vers le corps humain (ou face intérieure du matelas).

Le matelas de masse absorbante est, par conséquent, complètement inséré, exceptée la surface d'absorption dirigée vers le corps humain, dans une «poche» en matière imperméable, laquelle est aussi le revêtement extérieur de l'article. Une telle disposition renforce l'effet de «poche» sur toute l'épaisseur de la masse absorbante.

Toutefois cet article, suivant l'enseignement de l'art antérieur, est fixé au sous-vêtement au moyen d'une bande adhésive solidaire de la face extérieure, dans la zone médiane du matelas. Cette disposition ne peut éviter que la feuille perméable s'écarte, en utilisation, de la peau de l'utilisatrice, limitant de la sorte l'efficacité de la serviette.

Selon l'invention, la feuille extérieure imperméable présente des extensions latérales, obtenues en repliant ses parties latérales au-dessus des bords longitudinaux de la face du matelas dirigée vers le corps, lesdites extensions latérales constituent, de part et d'autre et hors de la surface délimitée par le matelas absorbant, des supports pour des bandes adhésives tel que des lignes de colle, continues ou interrompues, servant à la fixation de la serviette sur le sous-vêtement.

Une telle disposition des lignes de colle, hors de la surface délimitée par le matelas de masse absorbante, permet de maintenir en contact avec le corps la plus grande surface possible de matelas absorbant (la serviette est tendue latéralement).

Les lignes de colle sont disposées, de préférence à une distance des bords correspondants du matelas, sensiblement égale à l'épaisseur du matelas à l'état comprimé. Cette disposition offre l'avantage d'appliquer avec pression les bords longitudinaux de la serviette sur le corps de l'utilisatrice, et de renforcer, par conséquent, la sécurité (moins de risques de fuites latérales) et l'absorption (bon contact de la masse absorbante sur le corps de l'utilisatrice). De plus, la serviette périodique, maintenue au moyen de deux lignes de colle extérieures à la surface du matelas à tendance à s'incurver contre le corps de l'utilisatrice, ce qui renforce encore l'effet d'absorption.

D'autres avantages et caractéristiques de l'invention apparaîtront dans la description de deux exemples de réalisation, accompagnés d'un dessin dans lequel:

La figure 1 représente en section droite une serviette périodique conforme à la présente invention.

La figure 2 représente la même serviette périodique, en section droite, en position de fixation sur un sous-vêtement.

La figure 3 représente en perspective, une serviette périodique de même type, avec un matelas absorbant multicouches.

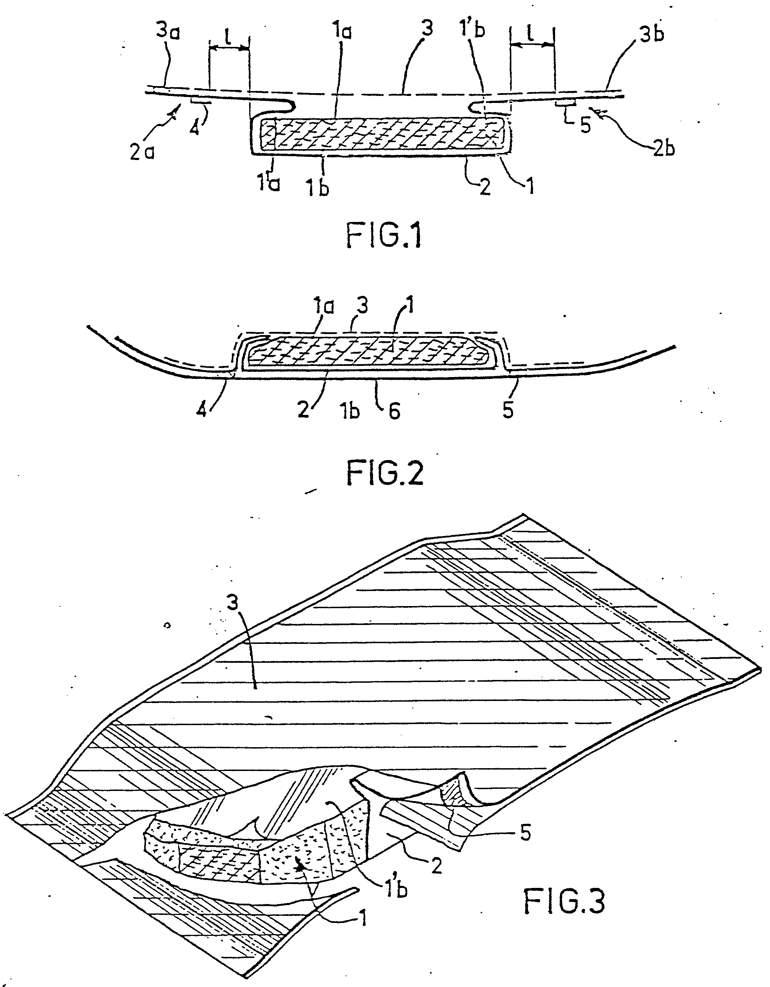

Sur le dessin des figures 1 et 2 est représentée une serviette périodique comportant un matelas absorbant 1, avec une face 1a dirigée vers le corps de l'utilisatrice et une face 1b, opposée. Le matelas absorbant 1 peut être, par exemple, un matelas de fibres cellulosiques ou matelas de «fluff».

Le matelas est enveloppé d'une feuille imperméable aux liquides 2, de préférence en matière synthétique (polyéthylène, par exemple), qui recouvre la face 1b du matelas, puis toute l'épaisseur dudit matelas et est également appliquée, par «retour» sur les bords longitudinaux 1'a et 1'b de la face du matelas dirigée vers le corps de l'utilisatrice. Le matelas absorbant 1 est, par conséquent, inséré dans une «poche» en matière imperméable, exceptée la plus grande partie de sa face dirigée vers le corps de l'utilisatrice, et qui constitue la surface d'absorption.

D'autre part, la feuille imperméable 2 est repliée sur elle-même au-dessus desdits «retours» sur les bords longitudinaux pour former des extensions latérales 2a et 2b qui s'étendent hors de la surface délimitée par le matelas absorbant.

Sur les extensions latérales 2a, 2b, sont déposées des bandes adhésives, respectivement 4 et 5, destinées à la fixation de la serviette sur un sous-vêtement symbolisé par la référence 6 sur le dessin de la figure 2.

Une feuille perméable aux liquides, référencée 3, revêt la partie supérieure de l'article d'hygiène.

Elle peut être fixée à la feuille imperméable 2 au moyen de lignes d'adhésif 3a, 3b, par exemple. Un tel agencement de la feuille perméable sur la feuille imperméable permet de maintenir la formation des replis de la feuille imperméable 2 sur les bords longitudinaux 1'a et 1'b de la face 1a du matelas.

Les bandes d'adhésif 4 et 5 pour le maintien en position de la serviette, sont situées à une distance «l» des bords respectifs du matelas absorbant, suffisante pour permettre la fixation sur le sous-vêtement 6, comme représenté sur le dessin de la figure 2.

De préférence, la distance «l» est choisie voisine de l'épaisseur du matelas comprimé.

La serviette est représentée en position d'utilisation sur le dessin de la figure 2. Compte-tenu de la disposition des bandes d'adhésif 4, 5, servant au maintien de la serviette, celle-ci se trouve maintenue sous tension transversale, cette tension ayant pour effet de garantir la plus grande surface de contact possible de la masse absorbante de la serviette périodique sur le corps de l'utilisatrice.

D'autre part, la disposition des bandes adhésives offre l'avantage de tendre la feuille perméable aux liquides, en contact avec le corps de l'utilisatrice, pour une meilleure absorption et une meilleure sécurité.

Sur le dessin de la figure 3, on a représenté la serviette périodique selon l'invention, avec un matelas absorbant constitué par deux nappes de «fluff», la nappe située près du corps étant moins comprimée que l'autre. On peut également introduire dans la masse du matelas absorbant des particules d'un produit absorbant fortement les fluides, de manière connue en soi.

Les lignes de colle, 4 et 5, sont avantageusement revêtues d'un papier protecteur à face siliconée, par exemple, de façon connue en soi.

## Revendications

1. Article d'hygiène pour l'absorption des liquides menstruels du type comprenant un matelas de matière absorbante (1) avec une face tournée vers le corps humain revêtue d'une feuille perméable aux liquides (3) et une face opposée revêtue d'une feuille extérieure (2) imperméable aux liquides qui enveloppe ledit matelas sur ladite face opposée (1b) sur toute l'épaisseur dudit matelas, puis, par retour, est appliquée sur les bords longitudinaux de la face du matelas (1a) dirigée vers le corps humain, caractérisé en ce que la feuille extérieure imperméable est repliée sur elle-même au-dessus de chaque bord longitudinal de manière à présenter des extensions latérales (2a, 2b) hors de la surface délimitée par le matelas absorbant (1), la feuille perméable aux liquides (3), étant fixée à la feuille (2) imperméable aux liquides sur les extensions latérales de cette dernière, et en ce que des bandes adhésives (4, 5) sont fixées sur la face des extensions latérales (2a, 2b) non dirigée vers le corps, le côté adhésif desdites bandes (4, 5) étant orienté à l'opposé de ladite face de manière à pouvoir adhérer à un sous-vêtement.

2. Article d'hygiène, selon la revendication 1, caractérisé en ce que les bandes adhésives (4, 5) sont situées à une distance «l» des bords respectifs du matelas absorbant, voisine de l'épaisseur du matelas comprimé.

3. Article d'hygiène selon l'une des revendications précédentes, caractérisé en ce que les bandes adhésives (4, 5) sont des lignes de colle, continues ou interrompues.

4. Article d'hygiène pour l'absorption des liquides menstruels selon la revendication 3, caractérisé en ce que les lignes de colle (4, 5) sont revêtues d'une bande protectrice, de préférence, d'une bande de papier à face siliconée.

## Claims

1. Article of hygiene for the absorption of menstrual fluids of the type comprising a pad of absorbent material (1) with one face turned towards the human body and covered with a sheet which is permeable to the fluids (3) and an opposite face covered with an outer sheet (2) which is impervious to the fluids and which encloses the said pad on the said opposite face (1b) over the entire thickness of the said pad, and then, by folding back, is applied onto the lengthwise edges of the face of the pad (1a) which is directed towards the human body characterized in that the impervious outer sheet is folded back onto itself above each lengthwise edge so as to have side extensions (2a, 2b) outside the area bounded by the absorbent pad (1), the sheet which is permeable to the fluids (3) being secured to the sheet (2) which is impervious to the fluids on the side extensions of the latter, and in that adhesive strips (4, 5) are secured to the face of the side extensions (2a, 2b) which is not directed towards the body, the adhesive side of the said strips (4, 5) being oriented away from the said face so as to be capable of adhering to an undergarment.

2. Article of hygiene, according to Claim 1, characterized in that the adhesive strips (4, 5) are situated at a distance «1» from the corresponding edges of the absorbent pad, which is similar to the thickness of the compressed pad.

3. Article of hygiene according to either of the preceding claims, characterized in that the adhesive strips (4, 5) are continuous or interrupted lines of adhesive.

4. Article of hygiene for the absorption of menstrual fluids according to Claim 3, characterized in that the lines of adhesive (4, 5) are covered with a protective strip, preferably, with a strip of paper with a silicone-treated face.

## Patentansprüche

1. Hygieneartikel zum Absorbieren von Menstruationsflüssigkeit, mit einer Schicht (1) aus absorbierendem Material, deren dem menschlichen Körper zugewandte Seite mit einer flüssigkeitsdurchlässigen Folie (3) und deren entgegengesetzte Seite mit einer flüssigkeitsundurchlässigen äusseren Folie (2) versehen ist, welche die Schicht auf der besagten gegenüberliegenden Seite (1b) über der gesamten Dicke der Schicht umhüllt und dann durch Umbiegen an den Längsrändern der dem menschlichen Körper zugewandten Seite der Schicht (1a) angebracht ist, dadurch gekennzeichnet, dass die undurchlässige äussere Folie auf sich selbst über jeden Längsrand so umgefaltet ist, dass sie seitliche Verlängerungen (2a, 2b) ausserhalb der von der absorbierenden Schicht (1) begrenzten Fläche besitzt, wobei die flüssigkeitsdurchlässige Folie (3) an der flüssigkeitsundurchlässigen Folie (2) auf den seitlichen Verlängerungen derselben befestigt ist, und dass Klebebänder (4, 5) auf der vom Körper abgewandten Seite der seitlichen Verlängerungen (2a, 2b) befestigt sind, wobei die klebende Seite der Klebebänder (4, 5) von der besagten Seite abgewandt sind, so dass sie an einem Unterbekleidungsstück haften kann.

2. Hygieneartikel nach Anspruch 1, dadurch gekennzeichnet, dass die Klebebänder (4, 5) in einem Abstand «l» von den entsprechenden Rändern der absorbierenden Schicht, benachbart zur Dicke der komprimierten Schicht angeordnet sind.

3. Hygieneartikel nach einem der vorhergehenden Ansprüche, dadurch gekennzeichnet, dass die Klebebänder (4, 5) kontinuierliche oder unterbrochene Klebelinien sind.

4. Hygieneartikel zum Absorbieren von Menstruationsflüssigkeit nach Anspruch 3, dadurch gekennzeichnet, dass die Klebelinien (4, 5) mit einem Schutzstreifen, vorzugsweise einem Papierstreifen mit silikonisierter Oberseite bedeckt sind.

0 074 984

FIG.1

FIG.2

FIG.3